# EUROPEAN PATENT APPLICATION

(11) **EP 4 691 353 A1**
(43) Date of publication of application: **11.02.2026**
(21) Application number: 24785406.0
(22) Date of filing: 01.04.2024
(51) Int. Cl.: A61B 5/021, A61B 5/024

(54) **CALIBRATION METHOD AND BLOOD PRESSURE ESTIMATION DEVICE FOR BLOOD PRESSURE ESTIMATION USING PHOTOPLETHYSMOGRAPHY SIGNALS**

(30) Priority: 07.04.2023 KR 20230046241
(71) Applicant: Sky Labs Inc., Gyeonggi-do 13486 (KR)
(72) Inventor: LEE, Min Hyung, Bucheon-si Gyeonggi-do 14600 (KR); SEOL, Ki Hyuk, Suwon-si Gyeonggi-do 16662 (KR); KIM, Chang Hyun, Seoul 05372 (KR); CHOI, Chang Woo, Uiwang-si Gyeonggi-do 16000 (KR); LEE, Byung Hwan, Yongin-si Gyeonggi-do 16981 (KR)
(74) Representative: Caldon, Giuliano
(86) International application number: PCT/KR2024/095650
(87) International publication number: WO 2024/210717

(57) **Abstract**

Disclosed are a calibration method for estimating blood pressure using a photoplethysmography signal and a blood pressure estimation apparatus using the same. More particularly, each step of the calibration method is performed by a blood pressure estimation apparatus using a photoplethysmography signal (PPG), the calibration method including: obtaining a measured value by controlling a sensor mounted therein for blood pressure estimation; determining a validity of the measured value; and calibrating a pre-stored blood pressure estimation algorithm based on the measured value, wherein the measured value includes a photoplethysmography signal and blood pressure value measured simultaneously.

## Description

### [Technical Field]

An embodiment of the present invention relates to a calibration method for estimating blood pressure using a photoplethysmography signal and a blood pressure estimation apparatus using the same, and more particularly, to a calibration method and blood pressure estimation apparatus that can improve estimation accuracy when estimating blood pressure using a photoplethysmography signal.

### [Background Art]

Recently, the prevalence of hypertension has been increasing due to the aging of society and the westernization of lifestyles and dietary habits resulting from the development of medical technology. Hypertension is a major indicator of kidney disease and serious cardiovascular diseases and is one of the most dangerous risk factors for death, so it essentially requires treatment and management.

For the prevention, recognition, and treatment of hypertension, the most important thing is to continuously measure blood pressure in daily life, but this is not being practiced.

To monitor blood pressure, a method of wearing a cuff and measuring blood pressure from pressure changes injected into the cuff has been typically used. The method using a cuff causes discomfort due to pressure during the measurement process and has a disadvantage in that it is not actually easy to carry because even portable products must include a cuff. The cuff-based method is not suitable for real-time blood pressure monitoring due to its disadvantages in terms of convenience and portability. Accordingly, research on measuring blood pressure without a cuff and without restraint is being actively conducted.

In particular, research using photoplethysmography (PPG) optical sensors has recently increased, because vascular elasticity information can be obtained using feature values, such as a percussion wave and a tidal wave, from the PPG signal. Since vascular elasticity information has a very high correlation with blood pressure, it can be used to estimate blood pressure.

Another reason for the many attempts to measure blood pressure through a PPG signal is that the PPG optical sensor has a very significant meaning as a personal cuff-less blood pressure measurement device. Recently, most wearable devices, such as smart bands and watches, are universally equipped with PPG optical sensors. This means that most wearable devices can have a blood pressure measurement function without adding a new sensor simply by installing a program. Being able to measure blood pressure through the PPG optical sensor of a wearable device may greatly improve the conventional blood pressure measurement method in terms of portability and convenience. In terms of convenience, wearable devices such as smart bands and smartwatches have the advantage that they are worn on the wrist and the user does not feel great discomfort. In summary, since blood pressure can be predicted using a PPG signal, the problems of convenience and portability of existing devices can be solved by applying it to wearable devices.

However, when measuring blood pressure without a cuff and without restraint, the measurement accuracy tends to decrease, requiring calibration technology.

However, when measuring blood pressure without a cuff and without restraint, the measurement accuracy tends to decrease, requiring calibration technology.

For example, blood pressure may be estimated by measuring Pulse Transit Time (PTT) using a PPG signal, but the measurement accuracy tends to decrease depending on real-time factors and algorithm differences in the method of calculating the time difference between P-peaks where the magnitude of the measured PPG's volume pulse wave is at its maximum.

As such, the calibration technology directly affects the accuracy of the measured value in cuffless blood pressure measurement.

### [Disclosure]

### [Technical Problem]

Therefore, the present invention has been made in view of the above problems, and it is one object of the present invention to provide a calibration method for improving the accuracy of a measured value during cuffless blood pressure measurement, and a blood pressure estimation apparatus for performing the method.

The problems to be solved by the present invention are not limited to the above-mentioned problem(s), and other unmentioned problem(s) will be clearly understood by those skilled in the art from the following description.

### [Technical Solution]

In accordance with an aspect of the present invention, the above and other objects can be accomplished by the provision of a calibration method, wherein each step of the calibration method is performed by a blood pressure estimation apparatus using a photoplethysmography signal (PPG), the calibration method including: obtaining a measured value by controlling a sensor mounted therein for blood pressure estimation; determining a validity of the measured value; and calibrating a pre-stored blood pressure estimation algorithm based on the measured value, wherein the measured value includes a photoplethysmography signal and blood pressure value measured simultaneously.

The measured value may be obtained a plurality of times at a first waiting time interval, and the calibration may be performed based on an average value of the plurality of obtained measured values.

The determining of the validity may include: determining whether a signal quality of a plurality of measured photoplethysmography signals is equal to or greater than a predetermined criterion, and determining the stability of a feature of a photoplethysmography signal measured during a first measurement time; and checking whether a difference between a plurality of input blood pressures is within a predetermined error range, wherein the first measurement time is within 30 seconds to 1 minute.

The obtaining of the measured value may include obtaining a first measured value and second measured value which are measured in physiologically different environments of a subject.

The different environments may refer to environments where a heart rate and a peripheral resistance are different from each other by a predetermined criterion or more.

The different environments may be environments in which user's activity states are different from each other, and the activity states may be a resting state and an active state.

The activity state may be obtained by analyzing a motion signal obtained from various motion sensors for detecting a movement of a subject.

The calibrating of the pre-stored blood pressure estimation algorithm may include: calibrating the pre-stored blood pressure estimation algorithm based on a difference between a blood pressure value of the first measured value and a blood pressure value of the second measured value according to a photoplethysmography signal of the first measured value and a photoplethysmography signal of the second measured value.

In accordance with another aspect of the present invention, provided is a blood pressure estimation apparatus, including: a biosignal measurement part for measuring a photoplethysmography signal (PPG); and a processor for estimating blood pressure by applying the photoplethysmography signal to a pre-stored blood pressure estimation algorithm, the blood pressure estimation apparatus further including: a communicator for obtaining a blood pressure value from an external device, wherein the processor determines the validity of the photoplethysmography signal and the obtained blood pressure value, and calibrates the pre-stored blood pressure estimation algorithm based on the photoplethysmography signal and the obtained blood pressure value.

The processor may determine whether a signal quality of a plurality of measured photoplethysmography signals is equal to or greater than a predetermined criterion, and determining the stability of a feature of a photoplethysmography signal measured during a first measurement time; and may check whether a difference between a plurality of input blood pressures is within a predetermined error range, wherein the first measurement time is within 30 seconds to 1 minute.

The blood pressure estimation apparatus may further include: a motion detection part including various motion sensors for detecting a movement of a subject; and an output part for displaying information for calibration or blood pressure estimation, wherein the processor analyzes a motion signal detected by the motion sensor to determine whether a user's activity state is a resting state or an active state.

The processor may control the output part to provide a guide for measuring blood pressure in different activity states.

The processor may calibrate the pre-stored blood pressure estimation algorithm based on a difference between a blood pressure value in an active state and a blood pressure value in a resting state, according to a photoplethysmography signal in the active state and a photoplethysmography signal in the resting state.

### [Advantageous effects]

According to the present invention, the reliability of blood pressure estimation using a photoplethysmography signal can be improved.

The effects of the present invention are not limited to the effects mentioned above, and other unmentioned effects will be clearly understood by those of ordinary skill in the art from the following description.

### [Description of Drawings]

FIG. 1 illustrates the block diagram of a blood pressure estimation apparatus according to an embodiment of the present invention.
FIG. 2 illustrates the block diagram of a blood pressure estimation apparatus according to another embodiment.
FIG. 3 illustrates the block diagram of the configuration of a processor according to an embodiment.
FIG. 4 illustrates the block diagram of the configuration of a processor according to another embodiment.
FIG. 5 illustrates a flowchart for explaining a method of performing a single calibration according to an embodiment.
FIG. 6 illustrates a flowchart for explaining a method of performing a multiple calibration according to an embodiment.
FIG. 7 illustrates a flowchart for explaining a blood pressure estimation method according to an embodiment of the present invention.

### [Best Mode for Implementing the Invention]

Specific details of other embodiments are included in the detailed description and the accompanying drawings. The advantages and features of the described technology and the method of achieving them will become clear by referring to embodiments described in detail below with reference to the drawings. Throughout the specification, the same reference numerals refer to the same components.

Terms such as 'first' and 'second' may be used to describe various components, but the components should not be limited by these terms. These terms are only used to distinguish one component from another. A singular expression includes a plural expression unless the context clearly indicates otherwise. Furthermore, when a part "includes" a component, it means that it may further include other components, not excluding them, unless there is a specific statement to the contrary. In addition, terms such as "...unit" and "module" described in the specification mean a unit that processes at least one function or operation, and this may be implemented as hardware, software, or a combination of hardware and software.

### [Description of Symbols]

100, 200 : blood pressure estimation apparatus
110 : biosignal measurement part
115 : motion detection part
120 : processor
211 : signal analysis part
222 : signal processing part
223 : feature extraction part
224 : calibration part
225 : blood pressure estimation part
231 : motion signal analysis part

### [Modes for Implementing the Invention]

Hereinafter, embodiments of a blood pressure estimation apparatus and method will be described in detail with reference to the drawings.

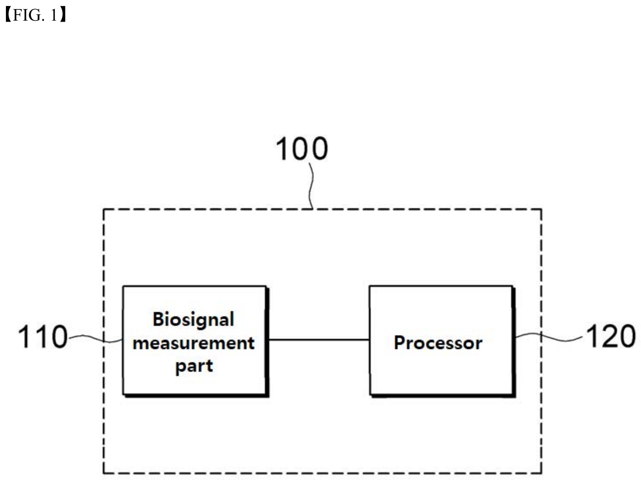

FIG. 1 illustrates the block diagram of a blood pressure estimation apparatus according to an embodiment of the present invention.

FIG. 1 illustrates the block diagram of the blood pressure estimation apparatus according to an embodiment. The blood pressure estimation apparatus 100 may be mounted in a terminal such as a smartphone, a tablet PC, a desktop PC, a notebook PC, or may be manufactured as an independent hardware device. Here, the independent hardware device may be a wearable device that may be worn by a subject, such as a wristwatch type, a bracelet type, a wristband type, a ring type, an eyeglass type, or a headband type, without being limited thereto.

Referring to FIG. 1, the blood pressure estimation apparatus 100 includes a biosignal measurement part 110 and a processor 120.

The biosignal measurement part 110 includes one or more sensors and may measure various biosignals from a subject through the sensors. Here, the sensors may be sensors that measure a photoplethysmogram (PPG), an electrocardiogram (ECG), a seismocardiogram (SCG), an electromyogram (EMG), a ballistocardiogram (BCG), and a pulse pressure, etc. However, it is not limited thereto.

The processor 120 may control the biosignal measurement part 110 when a user's blood pressure estimation request is received or when a blood pressure estimation criterion is satisfied. The processor 120 may receive a biosignal from the biosignal measurement part 110, and may estimate blood pressure or blood pressure change information by applying the received biosignal to a pre-stored blood pressure estimation algorithm. Here, the blood pressure change information may be the degree of change in blood pressure estimated by the blood pressure estimation algorithm performed multiple times. The blood pressure estimation algorithm and the blood pressure change information will be described in detail below.

The processor 120 may extract a feature that affects blood pressure from the biosignal. Here, the feature may include cardiac output (CO) and total peripheral resistance (TPR).

Here, the processor 120 may acquire an actual pulse pressure measured from a user as a feature related to pulse pressure through a pulse pressure sensor included in the biosignal measurement part 110 or an external pulse pressure measurement device. In addition, the processor 120 may extract a value corresponding to pulse pressure or a Heart Rate as a feature related to pulse pressure through Pulse Transit Time (PTT), Heart Rate Variability (HRV), peak amplitude analysis, etc., from biosignals such as PPG and BCG.

When the processor 120 extracts a feature from the biosignal, it may estimate blood pressure by applying the extracted feature to a pre-stored blood pressure estimation algorithm. For example, the processor 120 may acquire one or more characteristic points from the biosignal, and extract a feature for blood pressure estimation by combining the acquired characteristic points. At this time, the processor 120 may extract a one-cycle signal from a biosignal continuously measured for a predetermined time. In addition, a representative waveform with high representativeness may be obtained from the extracted one-cycle signal, or a representative waveform may be obtained by superimposing two or more one-cycle signals, and characteristic points may be obtained using the obtained representative waveform. At this time, a feature may be extracted by combining one or more of time information or amplitude information of the characteristic points.

The blood pressure estimation algorithm according to an embodiment may be obtained through regression analysis of a plurality of feature points extracted at equal time intervals from a waveform of a pulse wave signal and a blood pressure value. For example, the blood pressure estimation algorithm may be obtained through linear regression analysis, multiple regression analysis, nonlinear regression analysis, etc., on a plurality of feature points extracted at equal time intervals and a blood pressure value. Assuming that there is a relational expression between the plurality of extracted feature points and the blood pressure value, the relational expression may be obtained by analyzing a large amount of data, and the blood pressure estimation algorithm may include this relational expression.

A blood pressure estimation algorithm according to another embodiment may be obtained through machine learning on a plurality of feature points extracted at equal time intervals from a waveform of a pulse wave signal and a blood pressure value. For example, the blood pressure estimation algorithm may be obtained using an artificial neural network (ANN) algorithm, a k-nearest neighbor (k-NN) algorithm, a Bayesian network algorithm, a support vector machine (SVM) algorithm, a recurrent neural network (RNN) algorithm, etc. A network may be formed by setting at least one hidden layer between the plurality of extracted feature points and the blood pressure value (maximum and minimum blood pressures), and a hidden layer matrix may be obtained by repeatedly training a relational expression on this structure using a large amount of data. The blood pressure estimation algorithm may include this hidden layer matrix.

The blood pressure estimation algorithm according to an embodiment may be obtained using data collected from a randomly extracted population. That is, the blood pressure estimation algorithm may be obtained using data related to a pulse wave signal and blood pressure collected from a randomly extracted population.

A blood pressure estimation algorithm according to another embodiment may be obtained using data collected from a group classified according to a certain feature. For example, the blood pressure estimation algorithm may be obtained for each group classified by physical characteristics by using data related to a pulse wave signal and blood pressure collected from each group classified according to physical characteristics such as age, gender, weight, and height. As another example, the blood pressure estimation algorithm may be obtained for each group classified by situation by using data related to a pulse wave signal and blood pressure collected from each group classified according to situations such as a resting case or an active case.

A blood pressure estimation algorithm according to still another embodiment may be obtained using data collected from a user. For example, the blood pressure estimation algorithm may be obtained using data related to a pulse wave and blood pressure measured from a user. As another example, the blood pressure estimation algorithm may be obtained for each situation by using data related to a pulse wave signal and blood pressure measured from a user according to situations such as a resting case or an active case.

The blood pressure estimation algorithm may be obtained from an external device of a blood pressure estimation apparatus 200 and stored in the blood pressure estimation apparatus 200.

The blood pressure estimation algorithm according to an embodiment may be calibrated using a pulse wave signal and blood pressure value measured from a subject. For example, when the blood pressure estimation algorithm is obtained using data collected from a randomly extracted population, the blood pressure estimation algorithm may be calibrated using data related to a pulse wave and blood pressure measured from the user.

Data related to blood pressure for obtaining or calibrating the blood pressure estimation algorithm may be obtained using a general blood pressure measurement method.

A calibration according to an embodiment may be either a single calibration or a multiple calibration. A single calibration may be performed based on a biosignal measured without considering other conditions affecting the subject's blood pressure. A multiple calibration may use a multiple calibration performed based on biosignals measured under different conditions where at least one of a heart rate and a peripheral resistance, which are major factors affecting the subject's blood pressure, is different.

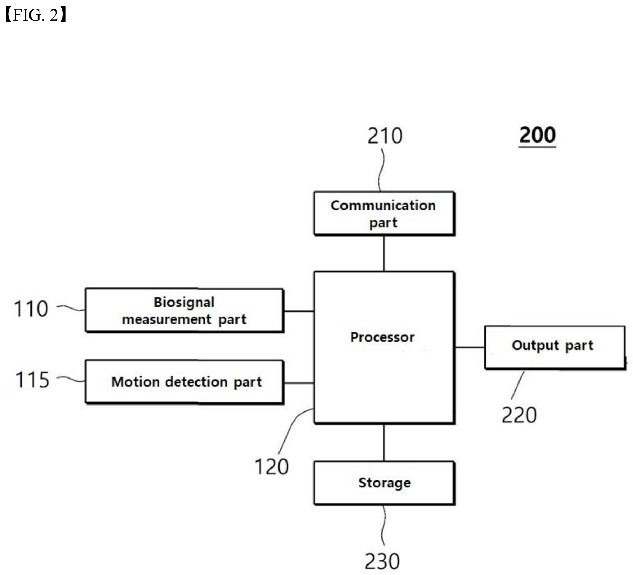

FIG. 2 illustrates the block diagram of a blood pressure estimation apparatus according to another embodiment.

Referring to FIG. 2, the blood pressure estimation apparatus 200 according to an embodiment may include a biosignal measurement part 110, a motion detector 115, a processor 120, a communication part 210, an output part 220 and a storage 230.

The biosignal measurement part 110 may be electrically connected to the processor 120 and may measure various biosignals from a user according to the control of the processor 120.

The motion detector 115 includes one or more sensors and may detect the movement of a subject through the sensors. Here, the sensors may include at least one sensor among a motion sensor, an acceleration sensor, a gyro sensor, and an angular velocity sensor.

The motion detector 115 may be electrically connected to the processor 120 and may measure the movement of the subject according to the control of the processor 120. For example, the motion detector 115 may detect whether a user is in an active state or a resting state.

The processor 120 receives a biosignal from the biosignal measurement part 110, and may extract features for blood pressure estimation, for example, a cardiac output feature and a total peripheral resistance feature, using the received biosignal.

In addition, the processor 120 receives a motion signal from the motion detector 115, and may estimate the subject's posture and life pattern using the received motion signal. For example, the processor 120 may analyze the motion signal to classify and record the subject's 'waking and sleeping times' and 'activity and rest times'.

According to this embodiment, the processor 120 may control the communication part 210 to transmit at least one of initial information, intermediate information, and final information for performing blood pressure estimation to an external terminal or device. The communication part 210 may establish a communication connection with an external terminal or device according to the control of the processor 120, and may receive an information request message requesting information transmission from the external terminal or device, or receive a blood pressure value for calibration according to the reception of the information request message or according to a preset time and period.

The communication part 210 may access a communication network using the wired/wireless communication technology according to the control of the processor 120. The communication part 210 may transmit the processing result of the processor 120 to an external device 250. Here, the communication technology may include Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, ultra-wideband (UWB) communication, Ant+ communication, WIFI communication, Radio Frequency Identification (RFID) communication, 3G communication, 4G communication, and 5G communication, etc, without being limited thereto.

Meanwhile, the processor 120 may guide a user to measure blood pressure for calibration through the output part 220. The output part 220 may output information for guiding a reference blood pressure measurement and/or a pulse pressure measurement according to the control of the processor 120 using a visual output module such as a display, an audio output module such as a speaker, or a haptic module for vibration or touch, etc. The processor 120 may output a user interface through the output part 220. A user may input the blood pressure and/or pulse pressure measured through an internal or external input device of the blood pressure estimation apparatus 100 using the user interface. Here, the external device may be any one of a smartphone, tablet PC, desktop PC, and notebook PC connected through the communication part, but is not limited thereto.

The processor 120 may store a biosignal measured through the biosignal measurement part 110 at the time of calibration, a feature extracted through the biosignal, and a blood pressure value obtained at the time of calibration, etc., as reference information for blood pressure estimation.

In addition, the storage 230 may store various information processed in the blood pressure estimation apparatus 200.

For example, the storage 230 may store various other reference information such as algorithms or programs executed within the blood pressure estimation apparatus 200, input/output biosignal data, user characteristic information related to a subject's physical information, a blood pressure estimation model, an offset estimation model, etc. The storage 230 may store data related to a pulse wave signal, body temperature, exercise state, etc., measured by the blood pressure estimation apparatus 200. The storage 230 may store at least one blood pressure estimation algorithm.

At this time, the storage 230 includes storage media such as a flash memory type, a hard disk type, a multimedia card micro type, a card type memory (e.g., SD or XD memory, etc.), Random Access Memory (RAM), Static Random Access Memory (SRAM), Read-Only Memory (ROM), Electrically Erasable Programmable Read-Only Memory (EEPROM), Programmable Read-Only Memory (PROM), a magnetic memory, a magnetic disk, and an optical disk, but is not limited thereto.

The processor 120 may extract a feature from a biosignal measured by the biosignal measurement part 110, and estimate blood pressure by referring to reference information at the time of calibration stored in the storage 230. At this time, when a heart rate and peripheral resistance, etc., are measured by the biosignal measurement part 110, the processor 120 may receive them through an interface of the communication part 210 or the output part 220, and may obtain an offset based on the received user's blood pressure. The processor 120 may correct an error between the cardiac output feature and total peripheral resistance feature extracted through the biosignal and the actual cardiac output and total peripheral resistance by using the offset obtained in this way.

When the blood pressure estimation by the processor 120 is completed, the output part 220 may output a biosignal, a blood pressure estimation result, and additional information according to the blood pressure estimation result. As an example, the output part 220 may visually provide various information to a user through a display module. For example, when displaying the blood pressure estimation result, if the estimated blood pressure is outside the normal range, a warning information may be displayed to the user by emphasizing it, for instance, by displaying it in red. As another example, various information may be provided to the user in a non-visual manner such as voice, vibration, or touch through a speaker module or a haptic module, etc. For example, it may provide guidance for systolic blood pressure and diastolic blood pressure by voice. At this time, if the estimated blood pressure is outside the normal range, it may inform the user of an abnormality in their health status through vibration or touch.

When the blood pressure estimation of the processor 120 is completed, the storage 230 may store the biosignal and/or the blood pressure estimation result, etc.

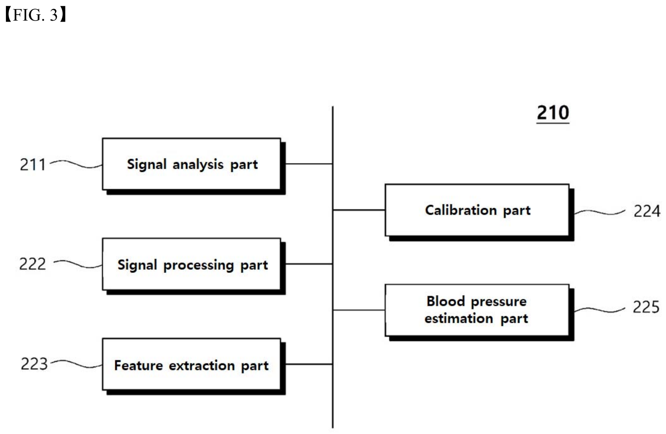

FIG. 3 illustrates the block diagram of the configuration of a processor according to an embodiment.

A processor described in FIG. 3 may be the processor 120 applied to FIG. 1 and FIG. 2.

The processor 120 may include a signal analysis unit 211, a signal processing unit 222, a feature extraction unit 223, a calibration unit 224, and a blood pressure estimation unit 225.

The processor 210 may include a signal analysis part 211, a signal processing part 222, a feature extraction part 223, a calibration part 224 and a blood pressure estimation part 225.

The signal analysis part 211 may determine whether a measured biosignal is a valid signal. To determine whether the measured biosignal is a valid signal, the signal analysis part 211 may check whether the signal quality of the measured biosignal is above a reference value and whether a feature of the biosignal is stable for a predetermined time.

For example, the signal analysis part 211 may determine the biosignal as a valid signal if a power spectrum value within a preset frequency range of the biosignal in a frequency domain is equal to or greater than a preset value, and a waveform of the biosignal is free of interruption or noise for a preset first time. This utilizes the fact that the components of the biosignal are mainly included in a low-frequency band in the frequency domain, and noise components are mainly included in a high-frequency band in the frequency domain. The preset frequency range may be, for example, 0.67 Hz to 3 Hz. As another example, the signal analysis part 211 may determine the biosignal as a valid signal if a difference between a maximum value and minimum value of the biosignal in a time domain is equal to or greater than a preset value.

When the pulse wave signal is determined to be a valid signal, the blood pressure estimation apparatus 200 may estimate a blood pressure value using the biosignal. When the biosignal is determined not to be a valid signal, in the blood pressure estimation apparatus 200, the processor 120 may control the biosignal measurement part 110 to measure the biosignal again.

The signal analysis part 211 may further include a noise filtering part (not shown) for removing a noise component included in the pulse wave signal.

In addition, the signal analysis part 211 may further include an amplification part (not shown) for amplifying the received biosignal to be suitable for signal processing.

In addition, the signal analysis part 211 may analyze a baseline for the biosignal and update the baseline.

Here, the baseline may be represented by a line connecting, for example, middle points between the highest and lowest points of each periodic waveform of the biosignal. The baseline may be in the form of a quadratic function, a cubic function, or a function having a plurality of inflection points. The signal analysis part 211 may update the baseline for the biosignal after checking the baseline of the biosignal.

In addition, the signal analysis part 211 may determine the validity of a blood pressure value obtained for calibration. The signal analysis part 211 may determine that the blood pressure values are valid if a difference between blood pressure values (respectively for SBP/DBP) obtained during a predetermined first time is within a predetermined error range.

The feature extraction part 223 may extract a characteristic point at a predetermined time interval for a one-cycle waveform of the biosignal from which a noise component has been removed, and extract a cardiac output feature and a total peripheral resistance feature by combining the extracted characteristic points and by combining various characteristic points obtained from the biosignal. For example, the feature extraction part 223 may extract a one-cycle waveform by checking the highest and lowest points for the signal from which the noise component has been removed. Next, the feature extraction part 223 may extract a characteristic point at a first time interval for the one-cycle waveform. For each cycle of the biosignal, a maximum blood pressure (cardiac systolic blood pressure) value and a minimum blood pressure (cardiac diastolic blood pressure) value may be obtained by applying the plurality of extracted feature points to the pre-stored blood pressure estimation algorithm.

Meanwhile, the cardiac output feature and the total peripheral resistance feature may be extracted by combining characteristic points differently according to the user's characteristics. In addition, the cardiac output feature and the total peripheral resistance feature may be respectively extracted for each type of blood pressure by combining characteristic points differently according to the blood pressure to be extracted, for example, mean blood pressure, diastolic blood pressure, and systolic blood pressure.

The feature extraction part 223 may extract a feature related to pulse pressure from the measured biosignal. For example, a Heart Rate (HR), etc., may be extracted as a feature related to pulse pressure through analysis of Pulse Transit Time (PTT), Heart rate variation (HRV), etc., of the measured biosignal.

The calibration part 224 may calibrate the pre-stored blood pressure estimation algorithm according to a single calibration technique by using the biosignal of the subject at a preset calibration time.

Single calibration is a technique that acquires a blood pressure value measured at the same time as a measured biosignal without considering the user's activity state, and performs calibration based on the biosignal and blood pressure value measured at the same time.

For example, the calibration part 224 may perform a single calibration according to a preset first condition. The first condition may be to perform the calibration multiple times at a preset time interval. The preset time interval may be, for example, within 30 minutes. This multiple-time calibration may be repeated every first period, for example, every month. Previous data may be updated based on the result value of a newly performed calibration.

According to this condition, for example, if the subject's systolic blood pressure measured in the first calibration is 120 mmHg at 1st and 124 mmHg at 2nd, and the subject's systolic blood pressure measured in the second calibration performed in the next cycle is, for example, 140 mmHg at 1st and 138 mmHg at 2nd, the baseline of the first calibration becomes 122 mmHg, and the baseline of the second calibration becomes 139 mmHg. In this case, the baseline may be updated by the new value of 139 mmHg obtained from the second calibration.

The blood pressure estimation part 225 acquires a blood pressure value or blood pressure change information by applying a plurality of specific points to the pre-stored blood pressure estimation algorithm using the pre-stored the blood pressure estimation algorithm.

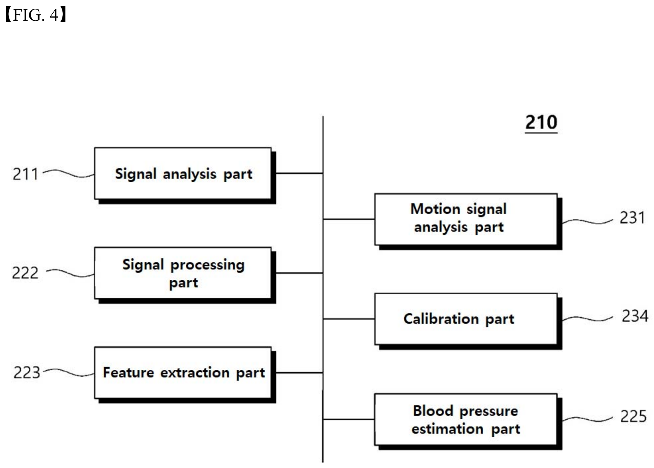

FIG. 4 illustrates the block diagram of the configuration of a processor according to another embodiment.

Since the processor described with reference to FIG. 4 is different from that of FIG. 3 only in the motion signal analysis unit 231 and the calibration part 234, the different components will be mainly described.

The motion signal analysis unit 231 estimates the activity state of a subject by analyzing a motion signal received from a motion detector (115 of FIG. 2). Here, the activity state may include a resting state and an active state. For example, if the motion detector (115 of FIG. 2) includes an acceleration sensor, it may receive an acceleration measurement value measured by the acceleration sensor for each time period, and determine the amount of movement based on the acceleration measurement value. The motion signal analysis unit 231 may estimate a life pattern by classifying a user's waking/sleeping time and activity/rest time based on an acceleration measurement value measured by the acceleration sensor during a preset period.

The calibration part 234 may calibrate the pre-stored blood pressure estimation algorithm according to a multiple calibration technique by using the biosignal of a subject according to a preset technique.

Multiple calibration is a technique that acquires measured values under physiologically different environments and performs calibration based on the acquired measured values. Here, the physiologically different environments refer to environments where at least one of a heart rate and a peripheral resistance, which are major factors affecting the subject's blood pressure, is different. The heart rate and peripheral resistance may differ depending on the user's activity state. For example, the calibration part 234 performs calibration based on a blood pressure value measured at the same time as a biosignal measured in consideration of the user's activity state. Here, for convenience of description, a biosignal and blood pressure value measured at the same time are referred to as a measured value. The activity state may include, for example, a resting state and an active state. Therefore, the pre-stored blood pressure estimation algorithm may be calibrated based on the measured value in the resting state and the measured value in the active state.

For example, if the subject's systolic blood pressure measured under a second condition is, for example, 120 mmHg at 1st and 124 mmHg at 2nd, and the subject's systolic blood pressure measured under a third condition physiologically different from the second condition is, for example, 140 mmHg at 1st and 138 mmHg at 2nd, a unique blood pressure change characteristic in the second condition and the third condition may be extracted.

When performing calibration, the calibration part 234 may provide a guide to the user for performing the calibration. For example, it may guide the user to contact the subject with the biosignal measurement part 110 for the measurement of a biosignal. In addition, it may guide the user on a measurement time or method that meets preset conditions, a communication connection between an external device and the communicator (210 of FIG. 2), etc. Here, the measurement time that meets the preset conditions may be an active period (e.g., immediately after waking up) and resting period (e.g., just before sleeping) estimated by the motion signal analysis part, but is not limited thereto.

If calibration is not performed according to the guide, the calibration part 234 may control the output part 220 to provide an alarm to induce calibration.

The blood pressure estimation algorithm may be calibrated by applying a result value from the calibration performed by the calibration part 234 to the blood pressure estimation algorithm.

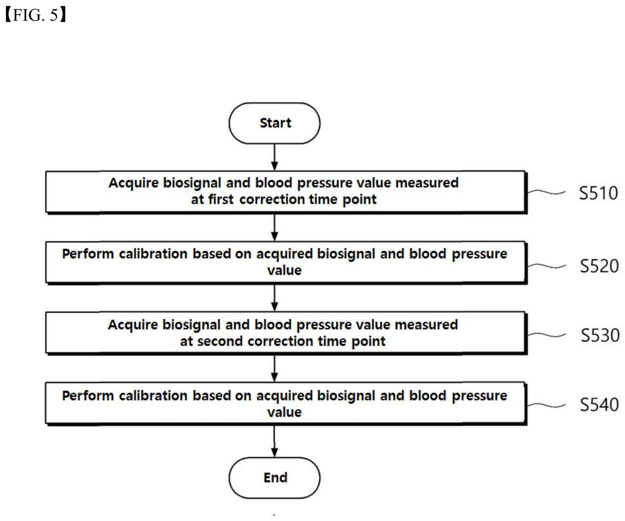

FIG. 5 illustrates a flowchart for explaining a method of performing a single calibration according to an embodiment.

In step 510, the blood pressure estimation apparatus 200 acquires a measured value by controlling a sensor mounted therein for blood pressure estimation.

A first measured value is obtained from a user at a first correction time. The measurement is performed for a preset first measurement time. Here, the predetermined first measurement time may be 30 seconds to 1 minute, etc., but may preferably be 40 seconds. However, it is not limited thereto. Here, the measured value includes a biosignal and blood pressure value measured at the same time as described above. In an embodiment, the first measured value may be an average value of measured values obtained multiple times. For example, the first measured value may be an average value of a measured value obtained at a first time point and a measured value obtained at a second time point. Although only the calculation of the average value of two measurements has been described in this example, the number of measurements is not limited to this and may be performed multiple times. However, the time between the first time point and the second time point should be within a predetermined first waiting time. The first waiting time may be about 30 minutes to 1 hour, but is not limited thereto.

In step 520, the validity of the measured value is determined.

For example, the validity of a biosignal is determined as valid if the signal quality of the biosignal measured multiple times is equal to or greater than a preset criterion, and the feature of the biosignal measured during the first measurement time is stable.

In addition, the blood pressure values are determined as valid if a difference between the blood pressure values input multiple times is within a predetermined error range.

In step S530, the pre-stored blood pressure estimation algorithm is calibrated based on the obtained first measured value.

Thereafter, the blood pressure estimation algorithm may be continuously calibrated by repeating steps S510 to S530.

For example, the blood pressure estimation apparatus 200 obtains a second measured value from the user at a second correction time by controlling a sensor mounted therein for blood pressure estimation. In an embodiment, the second measured value may be an average value of measured values obtained multiple times. The description of obtaining the average value of the measured values is omitted as it was described in step 510.

The first correction time and the second correction time may be preset, and may be, for example, 30-minute intervals, 1-hour intervals, 3-hour intervals, etc., but are not limited thereto.

The blood pressure estimation algorithm calibrated in step 520 is recalibrated based on the obtained second measured value.

In another embodiment, this single calibration may be performed periodically, for example, at one-month intervals.

As such, in single calibration, the calibration may be performed each time a measured value is obtained.

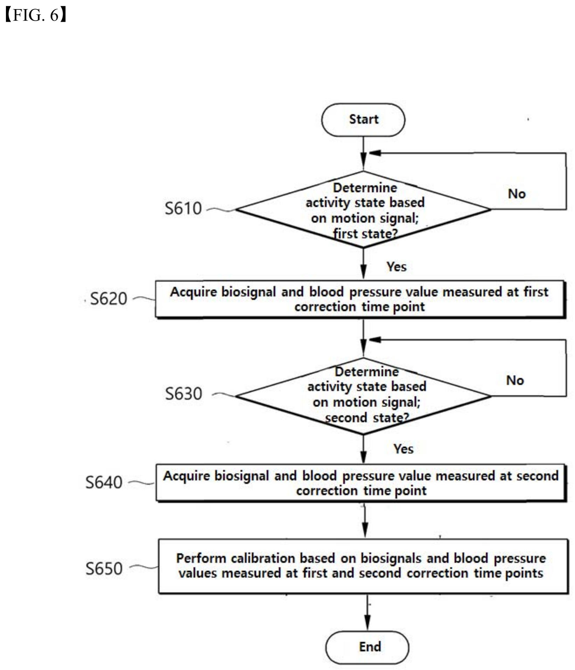

FIG. 6 illustrates a flowchart for explaining a method of performing a multiple calibration according to an embodiment.

In step 610, the blood pressure estimation apparatus 200 determines whether the user's activity state is a first state based on a motion signal.

When it is determined that the user's activity state is the first state, the process proceeds to step 620 to obtain a first measured value from the user by controlling a sensor mounted therein for blood pressure estimation. In an embodiment, the first measured value may be an average value of measured values obtained multiple times. For example, the first measured value may be an average value of a measured value obtained at a first time point and a measured value obtained at a second time point. Although only the calculation of the average value of two measurements has been described in this example, the number of measurements is not limited to this and may be performed multiple times. However, the time between the first time point and the second time point should be within a predetermined first waiting time. The first waiting time may be about 30 minutes to 1 hour, but is not limited thereto. Here, the measured value includes a biosignal and blood pressure value measured at the same time as described above.

In the next step 630, the blood pressure estimation apparatus 200 determines whether the user's activity state is a second state based on the motion signal. The first state and the second state are different activity states. For example, if the first state is a resting state, the second state becomes an active state, and if the first state is an active state, the second state may become a resting state.

If it is determined that the user's activity state is a second state, the process proceeds to step 640 to obtain a second measured value from the user by controlling a sensor mounted therein for blood pressure estimation. In an embodiment, the second measured value may be an average value of measured values obtained multiple times. The description of obtaining the average value of the measured values is omitted as it was described in step 620.

The first correction time and the second correction time may be preset, and may be, for example, 1-hour intervals, 8-hour intervals, 12-hour intervals, etc., but are not limited thereto.

In the next step 650, the pre-stored blood pressure estimation algorithm is calibrated based on the first measured value and the second measured value.

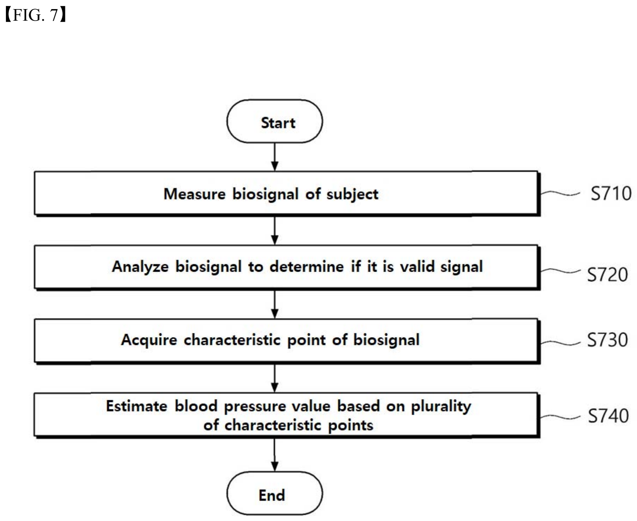

FIG. 7 illustrates a flowchart for explaining a blood pressure estimation method according to an embodiment of the present invention.

In step S710, the biosignal measurement part measures a biosignal of a subject.

The biosignal may be one or more of a photoplethysmogram (PPG), an electrocardiogram (ECG), a seismocardiogram (SCG), an electromyogram (EMG), a ballistocardiogram (BCG), and a pulse pressure, without being limited thereto.

In step S720, the processor may analyze the measured biosignal to determine whether it is a valid signal.

In step S730, the processor acquires a characteristic point of the measured biosignal. For example, the plurality of extracted feature points may be obtained from a waveform of the biosignal at equal time intervals.

In step S740, the processor estimates a blood pressure value by applying the plurality of characteristic points to the pre-stored blood pressure estimation algorithm.

The apparatus according to the embodiments may include a processor, a memory for storing and executing program data, a communication port for communicating with an external device, a user interface device such as a touch panel, a key, a button, etc.

Here, it will be understood that each block of the processing flowchart diagrams and combinations of the flowchart diagrams may be performed by computer program instructions. Since these computer program instructions may be loaded onto a processor of a general-purpose computer, a special-purpose computer, or other programmable data processing equipment, the instructions performed through the processor of the computer or other programmable data processing equipment create means for performing the functions described in the flowchart block(s). Since these computer program instructions may also be stored in a computer-usable or computer-readable memory that can direct a computer or other programmable data processing equipment to implement the function in a specific manner, the instructions stored in the computer-usable or computer-readable memory may also produce an article of manufacture containing instruction means for performing the functions described in the flowchart block(s). Since the computer program instructions may also be loaded onto a computer or other programmable data processing equipment, a series of operational steps may be performed on the computer or other programmable data processing equipment to create a computer-executed process, so that the instructions that execute on the computer or other programmable data processing equipment may also provide steps for executing the functions described in the flowchart block(s).

In addition, each block may represent a part of a module, segment, or code that includes one or more executable instructions for executing a specified logical function(s). Also, it should be noted that in some alternative execution examples, the functions mentioned in the blocks may occur out of order. For example, two blocks shown in succession may actually be performed substantially simultaneously, or the blocks may sometimes be performed in reverse order depending on the corresponding function.

Here, the term '...... part' used in this embodiment means software or a hardware component such as an FPGA or ASIC, and the '...... part' performs certain roles. However, '...... part' is not limited in meaning to software or hardware. A '...... part' may be configured to be in an addressable storage medium or may be configured to execute one or more processors. Therefore, as an example, a '...... part' includes components such as software components, object-oriented software components, class components, and task components, and processes, functions, attributes, procedures, subroutines, segments of program code, drivers, firmware, microcode, circuits, data, databases, data structures, tables, arrays, and variables. The functions provided within the components and '...... parts' may be combined into a smaller number of components and '...... parts' or further separated into additional components and '...... parts.' Furthermore, the components and '...... parts' may be implemented to execute one or more CPUs within a device or a secure multimedia card.

Those of ordinary skill in the art to which this specification pertains will understand that this specification may be embodied in other specific forms without changing its technical spirit or essential features. Therefore, the embodiments described above should be understood to be illustrative in all respects and not restrictive. The scope of this specification is indicated by the accompanying claims below rather than the detailed description above, and all changes or modified forms derived from the meaning and scope of the claims and their equivalent concepts should be interpreted as being included in the scope of this specification.

Meanwhile, although preferred embodiments of the present specification have been disclosed in this specification and the accompanying drawings and specific terms have been used, they are used only in a general sense to easily explain the technical content of this specification and to aid in the understanding of the invention, and are not intended to limit the scope of this specification. It is apparent to those of ordinary skill in the art to which this specification pertains that other modified examples based on the technical spirit of this specification are possible in addition to the embodiments disclosed herein.

## Claims

1. A calibration method, wherein each step of the calibration method is performed by a blood pressure estimation apparatus using a photoplethysmography signal (PPG),
the calibration method comprising:
obtaining a measured value by controlling a sensor mounted therein for blood pressure estimation;
determining a validity of the measured value; and
calibrating a pre-stored blood pressure estimation algorithm based on the measured value,
wherein the measured value comprises a photoplethysmography signal and blood pressure value measured simultaneously.

2. The calibration method according to claim 1, wherein the measured value is obtained a plurality of times at a first waiting time interval, and the calibration is performed based on an average value of the plurality of obtained measured values.

3. The calibration method according to claim 2, wherein the determining of the validity comprises:
determining whether a signal quality of a plurality of measured photoplethysmography signals is equal to or greater than a predetermined criterion, and determining a stability of a feature of a photoplethysmography signal measured during a first measurement time; and
checking whether a difference between a plurality of input blood pressures is within a predetermined error range, wherein the first measurement time is within 30 seconds to 1 minute.

4. The calibration method according to claim 1, wherein the obtaining of the measured value comprises obtaining a first measured value and second measured value which are measured in physiologically different environments of a subject.

5. The calibration method according to claim 4, wherein the different environments refer to environments where a heart rate and a peripheral resistance are different from each other by a predetermined criterion or more.

6. The calibration method according to claim 4, wherein the different environments are environments in which user's activity states are different from each other, and the activity states are a resting state and an active state.

7. The calibration method according to claim 6, wherein the activity state is obtained by analyzing a motion signal obtained from various motion sensors for detecting a movement of a subject.

8. The calibration method according to claim 4, wherein the calibrating of the pre-stored blood pressure estimation algorithm comprises:
calibrating the pre-stored blood pressure estimation algorithm based on a difference between a blood pressure value of the first measured value and a blood pressure value of the second measured value according to a photoplethysmography signal of the first measured value and a photoplethysmography signal of the second measured value.

9. A blood pressure estimation apparatus, comprising:
a biosignal measurement part for measuring a photoplethysmography signal (PPG); and
a processor for estimating blood pressure by applying the photoplethysmography signal to a pre-stored blood pressure estimation algorithm,
the blood pressure estimation apparatus further comprising: a communicator for obtaining a blood pressure value from an external device,
wherein the processor determines a validity of the photoplethysmography signal and the obtained blood pressure value, and calibrates the pre-stored blood pressure estimation algorithm based on the photoplethysmography signal and the obtained blood pressure value.

10. The blood pressure estimation apparatus according to claim 9, wherein the processor determines whether a signal quality of a plurality of measured photoplethysmography signals is equal to or greater than a predetermined criterion, and determining a stability of a feature of a photoplethysmography signal measured during a first measurement time; and checks whether a difference between a plurality of input blood pressures is within a predetermined error range, wherein the first measurement time is within 30 seconds to 1 minute.

11. The blood pressure estimation apparatus according to claim 9, further comprising:
a motion detection part comprising various motion sensors for detecting a movement of a subject; and
an output part for displaying information for calibration or blood pressure estimation,
wherein the processor analyzes a motion signal detected by the motion sensor to determine whether a user's activity state is a resting state or an active state.

12. The blood pressure estimation apparatus according to claim 11, wherein the processor controls the output part to provide a guide for measuring blood pressure in different activity states.

13. The blood pressure estimation apparatus according to claim 11, wherein the processor calibrates the pre-stored blood pressure estimation algorithm based on a difference between a blood pressure value in an active state and a blood pressure value in a resting state, according to a photoplethysmography signal in the active state and a photoplethysmography signal in the resting state.
